# EUROPEAN PATENT APPLICATION

(11) **EP 2 471 563 A1**
(43) Date of publication of application: **04.07.2012**
(21) Application number: 12159707.4
(22) Date of filing: 29.04.2005
(51) Int. Cl.: A61M 5/00, A61F 9/007

(54) **Apparatus and method for surgical enhancement of aqueous humor drainage**

(30) Priority: 29.04.2004 US 567024 P
(62) Divisional of application: 05742275.0
(71) Applicant: iScience Interventional Corporation, Menlo Park, CA 94025 (US)
(72) Inventor: Conston, Stanley, R., San Carlos, CA California 94070 (US); Hee, Michael, Burlingame, CA California 94010 (US); Kupiecki, David, J., San Francisco, CA California 94117 (US); McKenzie, John, San Carlos, CA California 94070 (US); Yanamoto, Ronald, San Francisco, CA California 94117 (US)
(74) Representative: Neilson, Martin Mark

(57) **Abstract**

An apparatus is provided for forming a tissue tract (8, 11 A, 17A) from within a first passageway of an eye (11, 17) connecting to a second passageway in the eye (12, 16) comprising an elongated tool with a proximal end and distal end. The tool has an outer diameter in the range of about 50 to about 1000 microns. Methods of using the tool are provided for creating a fluid path for aqueous humor of an eye from a first passageway of the eye, such as the Schlemm's Canal, to a second passageway, such as the suprachoroidal space.

## Description

### PRIORITY FROM RELATED APPLICATION

Priority is hereby claimed from U.S. Provisional Application Ser. No. 60/567,024, filed April 29, 2004, which is incorporated by reference in its entirety.

### BACKGROUND OF THE INVENTION

Glaucoma is a disease condition of the eye in which increased intraocular pressure (IOP) is created by dysfunction in the drainage mechanism for the aqueous humor. Aqueous humor is produced within the eye in the ciliary body and flows within the anterior region of the eye. The aqueous humor normally drains primarily through a network of tissues at the interior angle of the anterior chamber, named the trabecular meshwork and subsequently into a circular drainage space named Schlemm's Canal. The aqueous humor continues its drainage path into collector channels and finally into aqueous veins to enter the venous system. This pathway for aqueous humor drainage is often called the trabeculo-canalicular pathway. The aqueous humor also drains through a more diffuse secondary path through the scleral tissues, primarily from the suprachoroidal space and along the muscles and vascular vessels of the eye. This pathway for aqueous humor drainage is often called the uveal-scleral pathway and is believed to be responsible for 5 to 25 % of the total drainage of aqueous humor from the human eye.

Typically in glaucoma, the primary pathway for aqueous humor becomes narrowed or occluded, increasing IOP and resulting in gradual nerve damage and loss of vision. Such conditions are usually treated by topical drugs in the form of eye drops, but may result in surgical treatment if drug treatment becomes ineffective or if patient compliance is an issue. Traditional glaucoma surgery, such as trabeculotomy or trabeculectomy, involves dissection of the eye and the forming of a new flow passage through the trabecular meshwork into the anterior chamber. The fluid is channeled to a reservoir formed under the conjunctiva known as a bleb. While blebs are effective in removing the aqueous humor, blebs present a high incidence of post-surgical complications due to irritation and infection.

There is also a new class of surgical procedures which approach treatment of the ocular drainage system from the scleral tissues without penetrating the interior chamber of the eye. These procedures are termed "non-penetrating" surgery and involve careful surgical dissection of the sclera. Deep sclerectomy is a form of this procedure in which a portion of intrascleral tissue is removed nearly to Descemet's membrane to allow significant aqueous flow. Viscocanalostomy is another non-penetrating procedure in which the sclera is dissected to open Schlemm's Canal into an intra-scleral lake. Although non-penetrating procedures present fewer direct complications than traditional surgeries, most of the procedures still require extensive manual dissection of ocular tissues and often the subsequent formation of a subconjunctival bleb in order to provide an alternate drainage path for the aqueous fluid.

The present invention describes microsurgical tools and methods, which enable surgical creation of a tissue tract within the tissues of the eye to directly connect Schlemm's Canal to the suprachoroidal space, thereby forming a connection between the primary and secondary paths for aqueous humor drainage. By directing the flow of aqueous humor from the primary drainage pathway to the uveal-scleral pathway, restrictions in the primary pathway downstream of Schlemm's Canal or resistance due to increased episcleral venous pressure, may be circumvented. The tissue tract may also connect the anterior chamber to the suprachoroidal space to additionally bypass the trabecular meshwork and Schlemm's Canal. Since the aqueous humor passes directly into the secondary drainage pathway, the creation of a bleb is not required, eliminating the post-surgical complications associated with a bleb. Furthermore, the invention describes devices and materials that can be implanted in the tract to maintain the tissue space and fluid flow during the wound healing process. The tools and methods of the invention are designed for minimally invasive surgical use to minimize trauma and facilitate repeated treatment.

### SUMMARY OF THE INVENTION

An apparatus is provided for forming a tissue tract from within a first passageway of an eye connecting to a second passageway in the eye comprising an elongated tool with a proximal end and distal end, the tool having an outer diameter in the range of about 50 to about 1000 microns. The tool may comprise a flexible microcannula that may be located proximal to the distal end. The microcannula may have a rounded atraumatic distal terminus and a lubricious outer surface coating.

The tool may comprise an outer sheath and inner member. Preferably the outer sheath is flexible and the inner member has a higher flexural rigidity than the outer sheath.

The inner member may be removed and exchanged with another inner member during use of the apparatus in the eye.

The tool may comprise a mechanically cutting tip, and/or have the capacity to direct tissue ablative energy to the distal end. The distal end may be visible by medical imaging techniques or have an optical beacon visible by direct observation.

The apparatus may further comprise further a space-maintaining material or implant for placement within the tract. The material may comprise an anti-fibrotic agent and/or anti-thrombotic agent. The implant may be tube or stent-like device and may be made to be capable of changing configurations when delivered in the eye. The implant maintains the space of the tissue tract and provides a path for aqueous humor flow through the tissue tract. The implant may comprise microspheres, microparticles, microfibers, open or closed cell matrices, foam or gel. The implant may comprise a suture, a flange, or a tissue ingrowth surface to provide tissue fixation in the tract. The implant may be made of a permanent material such as, stainless steel, titanium, titanium alloy, cobalt chrome alloy, ceramic, carbon or polymeric material; or of a biodegradable or bioerodable material.

Methods are provided using the apparatus for creating a fluid path for aqueous humor of an eye from a first passageway of the eye, such as the Schlemm's Canal, to a second passageway, such as the suprachoroidal space, the method comprising:
a) inserting a microsurgical tool from a surgical access site into the first passageway;
b) advancing said tool along the first passageway to a desired site for formation of a tissue tract for the fluid path;
c) actuating the tool to form the tissue tract from the first passageway to the second passageway;
d) removing the tool and
e) closing the surgical access site.

The advancing of the tool and actuating may be performed a plurality of times to form multiple tissue tracts from the first passageway to the second passageway using a single surgical access site.

### DETAILED DESCRIPTION OF THE INVENTION

It has been found that Schlemm's Canal and the anterior edge of the suprachoroidal space are at similar depths from the scleral surface. The spatial relationship between the two passageways has been found to allow the natural geometry of the first passageway where the microsurgical tool is initially introduced to align the tool for creating a tissue tract to intersect the target passageway. Thus, we have found that a tool may be placed into the first passageway from a surgical access site and the tissue tract formed near the surgical access site. Alternatively, the tissue tract may be formed after advancement of the microsurgical tool along the first passageway. It has been found that a tool which is advanced into the first passageway for at least a short distance has a natural alignment with the second passageway, enabling tissue tracts to be formed without direct visualization at sites away from the surgical access site.

To access these passageways and to create the tissue tract, the tool will have an outer diameter in the range of about 50 to about 1000 microns.

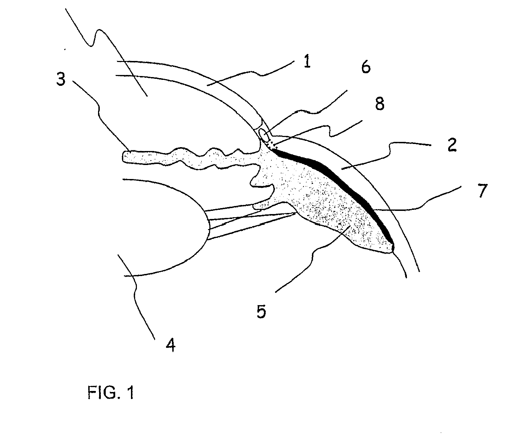

Referring to FIG.1, the relevant anatomical structures of the eye are shown. The cornea 1, anterior chamber 1A, sclera 2, iris 3, lens 4 and the ciliary body/choroid 5 are shown. The Schlemm's Canal 6 and suprachoroidal space 7 are shown connected by a tissue tract 8 made in accordance with the invention.

The invention provides apparatus, components thereof and related methods to surgically create a drainage tract for aqueous humor which connects the suprachoroidal space to Schlemm's Canal. Both the suprachoroidal space and Schlemm's Canal are passageways for aqueous humor drainage. In general, methods are provided for surgically accessing one or both of the two passageways and forming a tissue tract to connect to the passageways. Specifically, the invention involves the steps
a) inserting a microsurgical tool from a surgical access site into the first passageway;
b) advancing said tool along the first passageway to a desired site for formation of a tissue tract for the fluid path;
c) actuating the tool to form the tissue tract from the first passageway to the second passageway;
d) removing the tool and
e) closing the surgical access site.

Specifically, in one embodiment, the Schlemms's Canal is accessed through a surgical flap, small incision, or by penetration of a surgical tool. Then one or more microsurgical tools are inserted into the canal and advanced to create a tissue tract to the suprachoroidal space. Then, optionally an implant or material may be inserted to maintain the tract opening and fluid flow. Lastly, after the tools are withdrawn, the surgical access site is closed as required.

In an alternative embodiment, the procedure may be performed in a reverse manner by firstly accessing the suprachoroidal space of a living subject through a small incision or by penetration by a surgical tool, secondly inserting one or more microsurgical tools into the suprachoroidal space and advancing one or more tools to create a tissue tract that connects to Schlemm's Canal, thirdly to optionally insert an implant or material to maintain the channel opening and fluid flow, and lastly to close the surgical access site as required.

An alternative approach involves the steps of, firstly, performing a surgical flap cut-down to expose both Schlemm's Canal and a portion of the suprachoroidal space, creating a tissue tract that connects Schlemm's Canal and the suprachoroidal space, inserting an implant or material to maintain the tract opening and fluid flow, and lastly to close the surgical access site as required.

The invention provides apparatus to create a tissue tract within tissues of the eye such that the tract acts as a fluid path from Schlemm's Canal to the suprachoroidal space along with elements to implant into the tract to maintain the fluid flow path. Preferred apparatus are flexible microcatheter tools which may be advanced along the circumference of either Schlemm's Canal or the suprachoroidal space to allow minimally invasive surgery and to allow formation of multiple tissue tracts for drainage from a single surgical access site.

To practice the methods of the invention either Schlemm's Canal or the surprachoroidal space is accessed from the scleral surface. Both tissue regions are beneath the sclera varying in location from patient to patient in relation to surface landmarks of the eye. Both Schlemm's Canal and the suprachoroidal space may be identified by surgical dissection or high-resolution medical imaging methods such as high frequency ultrasound (HFU) or optical coherence topography (OCT). The use of medical imaging may be ideal in that the most surgically preferred access sites may be selected to minimize trauma and allow for use of minimally invasive surgical methods. The use of an ultrasound or optical contrast agent, either delivered directly to Schlemm's Canal, the suprachoroidal space or systemically to the subject, may facilitate identification. Pressure changes applied to the anterior chamber may also facilitate identification and selection of a preferred surgical access site.

The use of HFU or OCT is also desired to determine the optimal placement of the tissue tract. The surgeon may use imaging techniques to pre-plan the route of the surgery and to verify locations, direction and placement of tools and devices during the procedure. As an example, the surgeon may first access Schlemm's Canal and then create a tract toward and into the suprachoroidal space. Alternatively, the method may comprise access from the suprachoroidal space first and then through the eye tissues to either Schlemm's Canal or the anterior chamber.

The microsurgical tool may comprise an elongated tool with a distal tip that is first directed into either Schlemm's Canal or the suprachoroidal space. The tool may comprise a mechanically cutting tip such as a solid or hollow trocar-like member capable of creating a tunneled tract of controlled diameter through eye tissues. In an alternate embodiment, the tool may comprise means for blunt dissection, viscoelastic dissection or tissue penetration to form the tissue tract. In another embodiment, the tool may comprise a hollow tube with a sharpened distal edge used to core out a tissue tract. The removal or ablation of tissue to form the tract may aid in maintaining the tract and the subsequent placement of an implant or space-maintaining material into the channel.

Alternatively the tool may comprise a flexible outer sheath and inner member with the outer sheath disposed axially about the inner member. The inner member may comprise a trocar, solid rod, hollow rod or cylinder, needle, wire or optical fiber. Such an optical fiber may be used to carry visible light to the tip of the fiber, which may be disposed to reside at the tip of the sheath and hence may be used for direct visualization of the location of the tool through scleral tissues. In the case of an opaque outer sheath material, a cutout section or window near the distal tip of the sheath may be provided to visualize the optical fiber tip. The described optical beacon can provide an adjunct method of guiding the creation of the tract. Alternatively, the optical fiber may be used to carry energy for tissue ablation such as laser energy, in order to create the tract. The tip may also accommodate a radio frequency or thermal energy source to ablate tissue.

The microsurgical tool is sized for access into Schlemm's Canal and the suprachoroidal space and to create controlled diameter tissue tracts. Diameters from 50-1000 microns are useful, and in particular diameters from 100-500 microns are preferred for access to Schlemm's Canal. Outer diameter of a sheath member may correspond to these ranges and may comprise a wall thickness between 10 and 100 microns.

The microsurgical tool may act as a flexible microcannula or microcatheter to allow the distal tip to be advanced within Schlemm's Canal or the suprachoroidal space prior to forming the tissue tract. By forming the tissue tract away from the surgical access site, the stimulation of wound healing and scarring at the surgical access site should not interfere with patency of the tissue tract. Typically Schlemm's Canal and the suprachoroidal space are surgically accessed with an incision one to three millimeters around the circumference of the eye. To move one quarter the circumference or 3 clock hours, of the eye from the surgical access site, the microsurgical tool would be advanced approximately a minimum of 5 millimeters, thereby allowing the tissue tract to be formed sufficiently distant from the surgical access site. A rigid tool with the appropriate shape such as curvature to match the curvature of the tissue passageway, will enable 5 millimeters of advancement within the first tissue passageway. If a flexible microsurgical tool is used, the length of a flexible tool is preferred to be long enough to allow cannulation of at least one-half the circumference of Schlemm's Canal or the suprachoroidal space, approximately 22 to 40 mm. Flexible tools of such length allow the entire circumference of an eye to be treated at multiple sites from a single surgical access point.

In one embodiment, the microsurgical tool comprises a flexible outer sheath and an inner member with higher flexural rigidity than the outer sheath. The tool is inserted through a surgical access site into a first passageway of the eye, such as Schlemm's Canal or the suprachoroidal space. The inner member is removed or withdrawn from the distal tip of the tool to allow the flexible outer sheath to be advanced atraumatically within the passageway. After advancing the tip of the tool to a location distant from the surgical access site, the inner member is advanced within the outer sheath to the distal tip. This may the same of different inner member, since the inner member may be removed and exchanged with another during use. The now more rigid tool assembly may be used to advance into a second passageway of the eye, such as Schlemm's Canal, the suprachoroidal space, or the anterior chamber. The inner member may also accommodate cutting or tissue ablating components to facilitate formation of a tissue tract at the distal tip of the tool as previously described. The microsurgical tool may also incorporate features to aid atraumatic advancement within a tissue passageway such as a rounded atraumatic tip or a lubricious outer coating.

In creating the tissue tract with a microsurgical tool placed in Schlemm's Canal, the tract may be oriented radially outward from within the canal toward the suprachoroidal space. This approach would form the shortest length of tract to connect the two passageways. A tool advanced into the canal in this embodiment will preferably have a flexible tip so that it may be directed to form the tissue tract radially outward, orthogonal to the long axis of the tool. Alternatively, the tissue tract may be formed by advancing the tool tangentially from within the canal to intersect the suprachoroidal space. A tool used in this way will have the tissue cutting or ablation component for forming the tract at the distal tip of the tool in a forward facing direction aligned with the long axis of the tool.

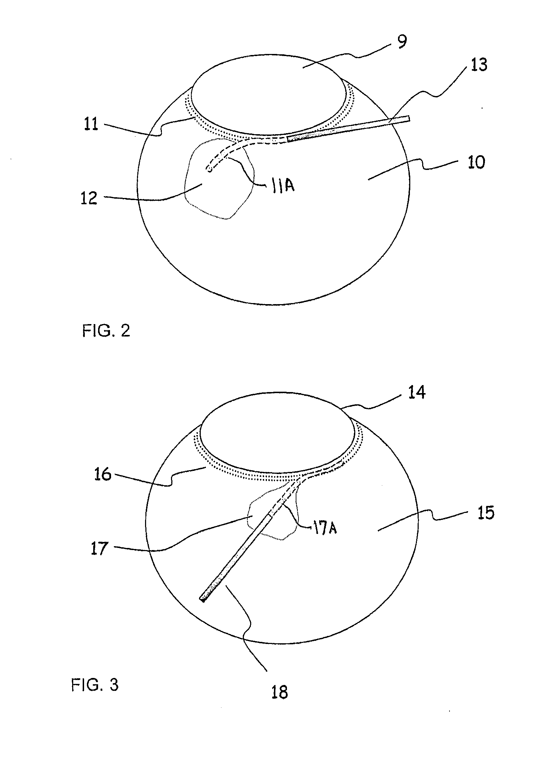

Preferring to FIG. 2, there is shown a diagram of a tissue tract 11A formed from the Schlemm's Canal 11 to the suprachoroidal space 12. The microsurgical tool 13 is inserted in to the canal 11 to create the tract. The cornea 9 and sclera 10 are also shown.

To exemplify a method of surgically creating a tissue tract for aqueous flow in the eye, the surgeon will access Schlemm's Canal and place a microsurgical tool within the canal. The microsurgical tool will comprise a sheath and trocar where the trocar has a distal tip configured to form a tissue tract. The tool is advanced within the canal to a location desired for the creation of a tissue tract. The tool is actuated to form the tissue tract connecting to the suprachoroidal space. A stent-like device attached to the tool is released to maintain the tract opening. The tool is removed and the surgical access site is then sealed by any requisite method.

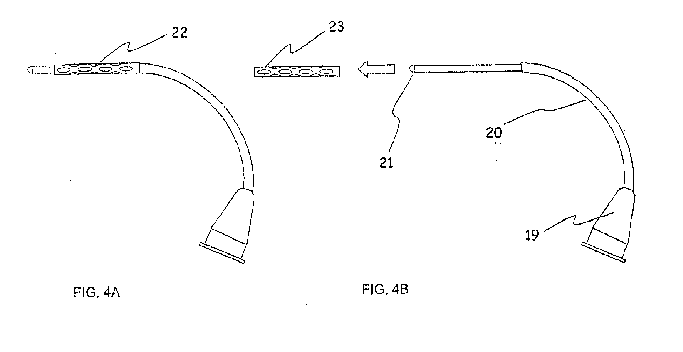

Referring to FIG. 4A-B, there is shown another microsurgical tool which may used in accordance with the invention. The tool has a Luer connector 19, flexible shaft 20 and an atraumatic tip 21 for advancement through tissue. The distal end of the tool accommodates a stent 22 secured to the tool. After formation of the tissue tract as described above, the stent is released within the tract where it remains as a stand alone delivered stent 23 in FIG 4B.

In creating a tract with a microsurgical tool first placed into the suprachoroidal space to form a tissue tract to connect to Schlemm's Canal the tract is radially oriented in an inward direction. A tool may be used aligned parallel with the equator of the eye having a flexible tip enable formation of the tract in a direction orthogonal to the long axis of the tool. Alternatively, the tool may be aligned in the suprachoroidal space at least partially directed toward Schlemm's Canal and the tissue tract formed by forward advancement of the tool. In an alternate embodiment, the microsurgical tool may be advanced from the suprachoroidal space toward Schlemm's Canal, and continued to be advanced until the tissue tract connects the suprachoroidal space to the anterior chamber. The tract may pass through Schlemm's Canal or may alternatively pass through the corneal scleral junction before entering the anterior chamber. To maximize aqueous outflow for the treatment of glaucoma, it may be advantageous in some patients to utilize this embodiment to form a fluid pathway from the anterior chamber to both Schlemm's Canal and the suprachoroidal space. As an example, the suprachoroidal space is surgically accessed and a tool placed within the space. A microsurgical tool comprising a sheath and trocar is used, wherein the trocar has a distal tip configured to form a tissue tract. The tool is advanced within the suprachoroidal space to a location desired for the creation of a tissue tract. The tool is actuated to form the tissue tract connecting to Schlemm's Canal. A stent-like device attached to the tool is released to maintain the tract opening. The tool is removed and the access site is then sealed by any requisite method.

In another example, the suprachoroidal space is surgically accessed and a tool placed within the space. A microsurgical tool comprising a sheath and trocar is used, wherein the trocar has a distal tip configured to form a tissue tract. The tool is advanced within the suprachoroidal space to a location desired for the creation of a tissue tract. The tool is actuated to form the tissue tract connecting to the anterior chamber either through Schlemm's Canal or in the region of the comeal-sclcral junction. A stent-like device attached to the tool is released to maintain the tract opening. The tool is removed and the access site is then sealed by any requisite method.

Referring to FIG. 3, there is shown a diagram of a tissue tract 17A connecting the suprachoroidal space 17 to Schlemm's Canal 16. The microsurgical tool 18 is inserted in to the suprachoroidal space 17 to create the tract. The cornea 14 and sclera 15 are also shown.

The microsurgical tool should preferable accommodate features to allow for orientation of the tract to be identified and controlled by the surgeon. The use of known medical imaging systems to coordinate or verify the position and orientation of the tract will aid accuracy and precision of tract placement. The imaging system should allow for identification of the tissue target and the tool position while minimizing the creation of artifacts into the image. Material selection and the use of contrast markers Known in the art of imaging may be utilized to provide the desired imaging properties for the tools.

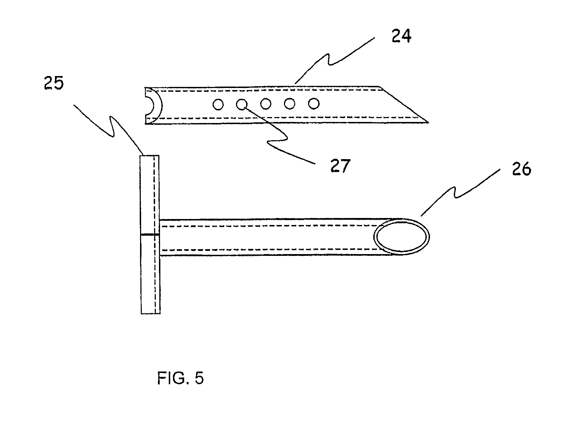

As previously described, the tract may optionally be filled with an implant to help maintain the patency and fluid flow of the tract. The implant may be especially advantageous when the tissue tract is formed by means in which tissue is not removed or ablated from the tract, such as by blunt dissection, viscoelastic dissection or penetration through an incision. The implant may also extend into the suprachoroidal space to maintain opening of the suprachoroidal space to aid fluid flow. Referring to FIG. 5, an implant 24 may have features such as a flange 25 to anchor one end into the anterior chamber or within Schlemm's Canal. A typical implant will have a beveled tip 26 to aid advancement within the tract and fenestrations 24 to aid in the distribution of fluid flow.

The implant may comprise an antifibrotic agent, space maintaining material, such as hyaluronic acid, tubular device, stent-like device or similar device to assure that the drainage tunnel remains patent. The implant may comprise permanent or biodegradable materials. Antifibrotic agents such as methotrexate, sirolimus, 5-fluorouracil and paclitaxel, may be applied or released from a device or implant within the tract. The implant may be in the form of microspheres, microparticles, microfibers, open-or closed-cell matrices, foams, gels, tube-like and stent-like devices, which may change their configuration in-situ after implantation. An implant device placed in the tract may comprise any suitable implant material, including metals, such as stainless steel, titanium, titanium alloys, cobalt-chrome alloys; a polymeric material; ceramics, and carbon materials such as vitreous carbon. The implant may also have surface porosity to encourage tissue ingrowth to provide mechanical fixation of the implant or mechanical features to facilitate suture fixation. Furthermore, a tubular device may incorporate multiple fluid outlets or fenestrations along its length to provide for improved flow characteristics. This is particularly important for an implant that resides in a tissue tract connecting the anterior chamber to both the suprachoroidal space and Schlemm's Canal since the tract would incorporate a flow path from the anterior chamber to both tissue passageways to maximize aqueous humor outflow.

An expandable stent-like implant may be placed within the tract to enlarge the tract diameter or provide fixation through mechanical means. The stent implant may be compressed and released within the tissue tract, or expanded in-situ, for example, with a balloon attached to the microsurgical tool. The stent implant may also incorporate shape memory functionality to allow it to expand once placed into the tissue tract. Furthermore, the microsurgical tool may be provided with an outer sheath that comprises the stent implant which is left behind after the tool core is removed.

The stent implant may be placed in a tissue tract formed between the suprachoroidal space and Schlemm's Canal or may alternatively be placed in a tissue tract formed between the suprachoroidal space and the anterior chamber. The stent implant may be pre-sized based on pre-surgical imaging or may be designed to be cut to size prior to or after implantation. The stent may also comprise a flange that is placed into Schlemm's Canal, the anterior chamber, or the suprachoroidal space to provide securement of the implant.

Furthermore, the implant can also be made to partially constrict the flow in the tract to provide a controlled amount of flow restriction that would be less than or equal to the maximum flow in the tract. This may be accomplished, for example, by making implants with different sized lumens, or varying amounts of fenestrations in the tube wall. Implants with different flow values may be created and chosen for optimization of aqueous flow by the surgeon. Also, the flow characteristics of a stent implant may be varied after the procedure upon examination of the patient's IOP. Various energy sources such as laser light, RF or microwave may be directed at a portion of the implant to dilate or contract discrete segments to control flow. A photoreactive polymer or a pre-stressed polymer similar to heat shrink tubing may be employed to perform this function.

The procedure may also be performed at more than site per eye as may be required to provide adequate drainage. In practice, the procedure may be performed on one or more sites, and the patient's IOP monitored post-surgically. If more pressure reduction is required, then a subsequent procedure may be performed at another target site. Multiple tissue tracts for aqueous humor drainage can thereby be created. The tracts may all be created in a single operation by advancing of the microsurgical tool and actuating it a plurality of times to form multiple tissue tracts from the first passageway to the second passageway using a single surgical access site.

The following examples are presented for the purpose of illustration and are not intended to limit the invention in any way.

### Example 1:

An enucleated human cadaver eye was used for this test. The eye was prepared by removing any excess tissues around the limbus and replacing lost fluid until the eye was firm to the touch. A rectangular scleral flap, approximately 5mm wide by 4mm long, was incised posterior from the limbus in order to expose Schlemm's Canal.

A flexible microcannula prototype with an outside diameter of approximately 220 microns was used. The microcannula was fabricated with a communicating element comprised of polyimide tubing 0.006 x 0.008" in diameter. Co-linear to the communicating element was a stiffening member comprised of a stainless steel wire 0.001" diameter and a plastic optical fiber 0.004" diameter. A heat shrink tubing of polyethylene terephthalate (PET) was used to bundle the elements into a single composite microcannula, The plastic optical fiber optic was incorporated to provide an illuminated beacon tip for localization. The beacon tip was illuminated using a battery powered red light laser diode source. The microcannula was inserted into the ostia of Schlemm's Canal and advanced along the canal. After advancement of approximately 3 clock hours along the canal the microcannula was advanced posterior into the suprachoroidal space, forming a tissue tract for flow of aqueous humor. The beacon tip of the microcannula was easily seen on the outside surface, through the scleral walls during the procedure, aiding guidance.

### Example 2:

An enucleated human cadaver eye was prepared as in Example 1. Using a high resolution ultrasound imaging system developed by the applicant, an imaging scan was made of the tissues to plan the access and route for placing a shunt from the suprachoroidal space to the anterior chamber. A radial incision was made at the pars plana and extending through the sclera to expose the choroid. A microcannula (MicroFil, World Precision Instruments, Sarasota, FL) was employed. The microcannula comprised of a 34 gauge fused silica core tube coated with polyimide. The microcannula was inserted into the surgical incision in an anterior direction and at a small angle with respect to the scleral surface. The microcannula was advanced until the distal tip had penetrated into the anterior chamber. High resolution ultrasound imaging confirmed the cannula placement in the suprachoroidal space and extending above the ciliary body and penetrating the anterior chamber at the anterior angle. The distal 15 mm of the cannula was then cut-off, remaining in place between the anterior chamber and the surgical site. Fluid flow was seen at the cannula proximal end. The proximal end was then placed into the suprachoroidal space and the incision sealed with cyanoacrylate adhesive.

A perfusion apparatus consisting of an elevated reservoir filled with phosphate buffered saline (PBS) connected to a 30 gauge hypodermic needle was used to perfuse the eye. The infusion pressure was set to a constant 10mm Hg by setting the height of the PBS reservoir. The needle was inserted through the cornea into the anterior chamber and the eye allowed to perfuse for 60 minutes to reach equilibrium. An injection of 0.1cc of methylene blue was made into the anterior chamber. The eye was perfused for another 4 hours. The perfusion was terminated and the eye examined visually. The scleral tissues were stained with methylene blue in an area around the suprachoroidal tract, demonstrating flow from the anterior chamber to the suprachoroidal space. A rectangular surgical flap was created around the previous radial incision with approximately 3 mm margins. The flap was retracted and the tissues observed. The inner scleral surface and the outer choroidal surface were uniformly stained with methylene blue demonstrating flow into the suprachoroidal space.

### Example 3:

A test was performed to evaluate a tubular implant connecting Schlemm's Canal to the suprachoroidal space. An enucleated human cadaver eye was used and prepared as in Example 1. A radial incision was made in the superio-temporal limbal region, the incision extending for approximately 4mm and to the depth of Schlemm's Canal. The posterior end of the incision was extended inward to expose the suprachoroidal without cutting into the choroid layer. The scleral spur fibers were left intact.

A tubular implant comprised ofpolyimide tubing, 0.0044" ID x 0.0050" OD was fabricated. The shunt tubing was split down the middle for a distance of 0.5mm. The two halves of the tube were then folded back to create a "Tee" shaped flange at the distal end. A bend of approximately 30° was made in the body of the tube, 0.75mm proximal to the split. The length of the base of the "Tee" was 2.5mm. The proximal end of the shunt was placed firstly into the SCS, then the distal end was placed into the Canal such that the flanges of the "Tee" were positioned inside the ostia of the cut-down, in order to stabilize the implant in-situ. The surgical incision was sealed with cyanoacrylate adhesive.

A perfusion apparatus consisting of an elevated reservoir filled with phosphate buffered saline (PBS), connected to a variable orifice flow meter and a 30 gauge hypodermic needle was used to perfuse and determine aqueous outflow in the eye. The contra lateral eye was prepared using a sham surgical procedure identical to the implanted eye, but without placement of the tubular implant. The infusion pressure was set to a constant 10mm Hg by setting the height of the PBS reservoir.

Perfusion was allowed to run for approximately 24 hours, at which time the aqueous outflow capacity of the test eye with the implant was higher than the control eye.

### Example 4:

A test as described in Example 3 was performed. After 24 hours of perfusion, methylene blue dye was injected into the anterior chamber of the test eye. After another 24 hour period, the methylene blue had cleared from the anterior chamber and there was visible evidence of staining of the suprachoroidal space, demonstrating flow from the anterior chamber.

### Example 5:

A test as described in Example 3 was performed. In this experiment, the dimensions of the tubular implant were increased. The dimensions of the implant were 0.0062" ID x 0.0080" OD. Perfusion at constant pressure of 10mm Hg was used. The perfusion was allowed to continue for 6 days and the aqueous outflow capacity of the test eye with the implant was higher than the control eye.

### Example 6:

A test as described in Example 3 was performed. The flanged tubular implant was fabricated from Pebax polymer with a durometer of 72 Shore D, and dimensions of 0.006" ID x 0.008" OD. The arms of the "Tee" were approximately 0.02" long and the base of the "Tee" was 0.2" long. The proximal end of the shunt was beveled and small fenestrations were made along the length of the base of the "Tee" for better distribution of fluid flow. The implant is similar to that shown in FIG. 5

## Claims

1. An implant for placement in a surgically formed tissue tract in the eye between the suprachoroidal space and Schlemm's Canal, wherein said implant maintains the space of said tissue tract and provides a path for aqueous humor flow through said tissue tract.

2. The implant according to Claim 1 comprising microspheres, microparticles, microfibers, open or closed cell matrices, foam, gel, a tubular device, or a stent-like device.

3. The implant according to Claim 1 comprising a suture, a flange, or a tissue ingrowth surface to provide tissue fixation of said implant in said tract.

4. The implant according to Claim 1 comprises stainless steel, titanium, titanium alloy, cobalt chrome alloy, ceramic, carbon or polymeric material.

5. The implant according to Claim 1 comprising a biodegradable or bioerodable material.

6. The implant according to Claim 1 comprising an anti-fibrotic material.
